# FASCICULE DE BREVET EUROPEEN

(11) **EP 1 169 057 B1**
(45) Date de publication et mention de la délivrance du brevet: **27.10.2004**
(21) Numéro de dépôt: 00918952.3
(22) Date de dépôt: 12.04.2000
(51) Int. Cl.: A61K 39/21, C07K 14/16

(54) **VACCIN ANTI-VIH-1 COMPRENANT TOUT OU PARTIE DE LA PROTEINE TAT DE VIH-1**
ANTI-HIV-1 IMPFSTOFF ENTHALTEND DAS GANZEN HIV-1 TAT PROTEIN ODER EIN TEIL DAVON
ANTI-HIV 1 VACCINE COMPRISING THE ENTIRE OR PART OF THE TAT HIV-1 PROTEIN

(30) Priorité: 13.04.1999 FR 9904610; 29.12.1999 FR 9916633
(43) Date de publication de la demande: 09.01.2002
(73) Titulaire: CENTRE NATIONAL DE LA RECHERCHE SCIENTIFIQUE, 75016 Paris Cédex (FR)
(72) Inventeur: LORET, Erwann, F-13009 Marseille (FR)
(74) Mandataire: Warcoin, Jacques
(86) Numéro de dépôt international: PCT/FR2000/000938
(87) Numéro de publication internationale: WO 2000/061067

(56) Documents cités:
- WO-A-96/27389
- DE-A- 19 514 089
- US-A- 5 889 175
- US-A- 5 891 994
- GOLDSTEIN G: "HIV - 1 Tat protein as a potential AIDS vaccine." NATURE MEDICINE, (1996 SEP) 2 (9) 960-4. REF: 34, XP002129594
- PELOPON ESE J M JR ET AL: "Full peptide synthesis, purification, and characterization of six Tat variants. Differences observed between HIV-1 isolates from Africa and other continents." JOURNAL OF BIOLOGICAL CHEMISTRY, (1999 APR 23) 274 (17) 11473-8., XP002129596
- LEFEVRE E A ET AL: "Cutting edge: HIV-1 Tat protein differentially modulates the B cell response of naive, memory, and germinal center B cells." JOURNAL OF IMMUNOLOGY, (1999 AUG 1) 163 (3) 1119-22., XP002129595
- CASELLI E ET AL: "DNA immunization with HIV - 1 tat mutated in the trans activation domain induces humoral and cellular immune responses against wild-type Tat." JOURNAL OF IMMUNOLOGY, (1999 MAY 1) 162 (9) 5631-8., XP002129597
- T. HUET ET AL.: "A HIGHLY DEFECTIVE HIV-1 STARIN ISOLATED FROM A HEALTHY GABONESE INDIVIDUAL PRESENTING AN ATYPICAL WESTERN BLOT" AIDS, vol. 3, 1989, pages 707-715, XP000867752 cité dans la demande
- H. LE BUANEC ET AL.: "A PROPHYLACTIC AND THERAPEUTIC AIDS VACCINE CONTAINING AS A COMPONENT THE INNOCUOUS TAT TOXOID" BIOMED & PHARMACOTHER, vol. 52, 1998, pages 431-435, XP000867754 cité dans la demande
- GREGOIRE C J ET AL: "Conformational heterogeneity in two regions of TAT results in structural variations of this protein as a function of HIV-1 isolates." JOURNAL OF BIOLOGICAL CHEMISTRY, (1996 SEP 13) 271 (37) 22641-6., XP002129593 cité dans la demande
- DATABASE BIOSIS [en ligne] BIOSCIENCES INFORMATION SERVICE, PHILADELPHIA, PA, US; 1995 RE M C ET AL: "Effect of antibody of HIV-1 tat protein on viral replication in vitro and progression of HIV-1 disease in vivo." Database accession no. PREV199698593925 XP002148917 & JOURNAL OF ACQUIRED IMMUNE DEFICIENCY SYNDROMES AND HUMAN RETROVIROLOGY, vol. 10, no. 4, 1995, pages 408-416, ISSN: 1077-9450
- DATABASE BIOSIS [en ligne] BIOSCIENCES INFORMATION SERVICE, PHILADELPHIA, PA, US; 1990 BRAKE D A ET AL: "CHARACTERIZATION OF MURINE MONOCLONAL ANTIBODIES TO THE TAT PROTEIN FROM HUMAN IMMUNODEFICIENCY VIRUS TYPE 1" Database accession no. PREV199089070184 XP002148918 & JOURNAL OF VIROLOGY, vol. 64, no. 2, 1990, pages 962-965, ISSN: 0022-538X
- OPI ET AL: 'Tat HIV-1 Primary and Tertiary Structures Critical to Immune Response Against Non-Homologuous Variants' JOURNAL OF BIOLOGICAL CHEMISTRY vol. 277, no. 39, 27 Septembre 2002, pages 35915 - 35919
- THIKONOV ET AL: 'Tat-Neutralizing Antibodies in Vaccinated Macaques' JOURNAL OF VIROLOGY vol. 77, no. 5, Mars 2003, pages 3157 - 3166
- OPI ET AL: 'Full-length HIV-1 Tat protein necessary for a vaccine' VACCINE 2004,
- GRINGERI ET AL: 'Safety and Immunogenicity of HIV-1 Tat Toxoid in immunocompromised HIV-1-infected patients' JOURNAL OF HUMAN VIROLOGY vol. 1, no. 4, 1998, pages 293 - 298
- LE BUANEC ET AL: 'Procedure for preparing biologically inactive, but immunogenic HIV-1 Tat protein (Tat Toxoid) for human use' BIOMED & PHARMACOTHERAP vol. 54, 2000, pages 41 - 44

## Description

La présente invention a pour objet un vaccin anti-VIH-1 comprenant tout ou partie de la protéine Tat de VIH-1 ainsi que la préparation d'anticorps monoclonaux ou polyclonaux aptes à reconnaître plusieurs variants de Tat et un procédé de détection de Tat dans un échantillon biologique.

L'objet de la présente invention concerne l'utilisation d'une protéine de régulation du virus de l'immunodéficience humaine (VIH), la protéine Tat, comme vaccin contre ce virus ainsi que la mise en évidence de cette protéine chez des individus infectés par le VIH.

Tat est une protéine virale essentielle pour l'expression des gènes viraux et la réplication du virus VIH-1.Le gène Tat est composé de deux exons codant pour une protéine de 99 à 106 résidus en fonction des isolats. Cependant une activité Tat avec des formes à 86 résidus ayant une partie de l'extrémité C terminale délétée a déjà été décrite. Ces dérivés Tat de 86 résidus seraient des artéfacts de laboratoire ou des formes ancestrales qui n'existent plus à l'état naturel. Cette protéine permet l'activation des gènes du VIH-1 grâce à sa fixation sur une cible nucléotidique appelée TAR localisée à l'extrémité 5' des ARNm de VIH-1. Elle est sécrétée par les cellules infectées par le VIH et est indispensable pour une transcription réverse efficace de VIH-1. Une fois à l'extérieur de la cellule, elle est capable d'activer des cellules infectées éloignées et d'induire l'immunodéficience de cellules T non infectées. En outre, elle est directement impliquée dans des pathologies liées au SIDA, comme le sarcome de Kaposi.

La mise au point du vaccin contre le SIDA est très attendue au niveau planétaire mais l'obtention d'un vaccin contre le SIDA présente deux difficultés majeures. La première est liée à l'extrême variabilité de la protéine d'enveloppe GP120. La deuxième difficulté est due au fait que des anticorps anti-GP120 semblent aggraver la maladie pour des patients ayant un SIDA déclaré. Toutefois des résultats préliminaires indiquent qu'un effet protecteur pourrait être possible avec des anticorps anti-GP120 chez des personnes n'étant pas contaminées au préalable. Une phase II avec un vaccin anti-GP120 a été lancée par la société Roche aux Etats Unis. Les cohortes sélectionnés pour cette phase II sont bien suivis au niveau médical. D sera toutefois difficile d'obtenir une surveillance médicale équivalente lorsque le vaccin sera commercialisé.

Avec la GP120, Tat est détectée dans le sang de patients contaminés par le VIH-1. Les macrophages et lymphocytes T cytotoxiques (LTC) chargés d'éliminer toutes les cellules contaminées par un virus ont leur activité progressivement bloquée par Tat, qui agit donc comme un immunosuppresseur.. Des anticorps anti-Tat (ou des principes actifs ciblant Tat) devraient permettre la restauration de l'activité des LTC et des macrophages.

Tat est donc une cible privilégiée aussi bien pour le développement d'antiviraux anti Tat que pour une approche vaccinale.

DE-A-195 14 089 et US 5,891,994 concernent la préparation d'anticorps par immunisation d'un animal avec un fragment de la protéine Tat. L'intérêt potentiel que pourrait avoir la protéine Tat de HIV-1 dans un vaccin contre le Sida a également été décrit en 1996 par Goldstein (Nature Médecine) ; ladite protéine Tat comprenant cependant 72 acides aminés.

La protéine Tat a donc déjà fait l'objet d'expérimentations. En particulier, des essais précliniques, obtenus avec une protéine Tat recombinante biologiquement inactive (Tat toxoïde), ont montré que Tat toxoïde entraîne, chez les patients séronégatifs une forte et persistante production d'anticorps anti-Tat (Le Buanec et al., 1998). Chez des patients séropositifs et immunodéficients une augmentation significative du taux d'anticorps anti-Tat est observé par rapport au taux normal d'anticorps anti-Tat (Westendorp et al., 1995).

Cette Tat toxoïde est une protéine recombinante qui est rendue biologiquement inactive après la carboxyméthylation des cystéines de Tat. Les inventeurs ont pu produire également une Tat carboxyméthylée (Tat Bru cmC) et ils ont observé également une perte de l'activité de transactivation alors que le même variant Tat avec les cystéines libres (Tat Bru fC) est capable de transactiver. Tat Bru cmC est capable de se fixer sur TAR avec une affinité comparable à Tat Bru fC.

Un inconvénient majeur dans l'utilisation comme vaccin de Tat carboxyméthylée est que la modification chimique des cystéines entraîne des changements conformationnels que les inventeurs ont observé par dichroïsme circulaire et RMN. Bien que des changements conformationnels existent parmi les différents variants Tat, la carboxyméthylation des cystéines entraîne des modifications majeures dans le repliement de la chaîne peptidique de Tat qui, de ce fait, n'est pas un bon candidat pour la préparation d'un vaccin anti-Tat.

Il convient par conséquent d'utiliser dans un vaccin anti-VIH-1 une protéine Tat défectueuse mais possédant les caractéristiques structurales similaires aux Tat fonctionnelles. Cette Tat devrait en effet présenter une structure tridimensionnelle la plus proche possible des protéines Tat naturelles, de façon à être capable d'induire une réponse immunitaire similaire à celle induite par les Tat naturelles, mais ne pouvant pas transactiver, c'est-à-dire, comme précédemment indiqué, incapable d'activer des cellules infectées éloignées et induire l'immunodéficience de cellules T non infectées. Il est en effet crucial d'inhiber cette immunodéficience. Caselli et al. (1999, Journal of Immunology) ont d'ailleurs montré qu'il était possible d'obtenir une réponse immune cellulaire et humorale contre la protéine Tat de type sauvage à la suite d'une immunisation au moins de gènes *tat* mutés dans le domaine de transactivation.

La présente invention a pour objet un vaccin anti-VIH-1 comprenant une protéine Tat de VIH-1 comprenant entre 99 et 106 acides aminés, capable de se fixer sur TAR et incapable de transactiver, en association avec un véhicule pharmaceutiquement acceptable et, le cas échéant, un ou plusieurs adjuvant(s) de l'immunité approprié(s). Cette protéine Tat doit être capable de générer, chez un animal chez qui cette protéine serait administrée, une production d'anticorps capables de reconnaître d'autres variants de cette protéine et selon un taux suffisant pour inhiber la transactivation desdits variants.

Ainsi, non seulement une protéine Tat peut être utilisée pour la préparation d'un vaccin conforme à l'invention mais également seulement une ou plusieurs partie(s) de cette protéine susceptible(s) de remplir les fonctions recherchées pour la protéine Tat dans le cadre de la présente invention. Le susdit vaccin peut également comprendre une combinaison de différentes protéines Tat ou encore des parties de protéines Tat différentes.

Selon un mode de réalisation avantageux de l'invention, la protéine Tat, comprend de préférence 101 acides aminés.

La protéine Tat susceptible d'être utilisée dans le vaccin conforme à l'invention doit donc être différente des protéines Tat fonctionnelles dites « naturelles », c'est-à-dire extraites de différentes souches de VIH-1 présentes à l'état naturel, de façon à être incapable de transactiver tout en se fixant sur TAR. C'est la raison pour laquelle, les Tat utilisées dans le vaccin conforme à l'invention présentent une séquence nucléotidique ayant au moins une mutation par rapport à la séquence nucléotidique d'une Tat fonctionnelle. Cette mutation, généralement ponctuelle, peut par exemple donner lieu à la suppression, l'ajout ou substitution d'un ou plusieurs acide(s) aminé(s).

Dans le cadre de leurs travaux, les inventeurs ont tout d'abord étudié le variant Tat correspondant à l'isolat Bru (86 résidus) puis ont synthétisé cinq autres variants représentatifs chacun de la diversité structurale observée avec cette protéine (Grégoire et Loret, 1996) : Tat Z2 (86 résidus), Tat Mal (87 résidus), Tat Eli (99 résidus), Tat Oyi (101 résidus) et Tat Jr (101 résidus). 6 groupes structuraux parmi tous les isolats de VIH-1 ont ainsi été déterminés en fonction de la taille des protéines et de la nature de leurs mutations. Il était vraisemblable que tous ces variants avaient des activités pharmacologiques similaires à Tat Bru qui a été synthétisée deux fois, avec les cystéines carboxyméthylées (Tat Bru cmC) et ensuite avec les cystéines libres (Tat Bru fC). La chimie utilisée est de type fastFmoc avec l'activateur HBTU. La purification des protéines a été effectuée par chromatographie liquide haute performance. Il s'est avéré que toutes ces protéines synthétiques sont capables de se fixer sur TAR mais de grandes disparités se sont révélées dans le test de transactivation sur cellule HeLa. Le résultat le plus surprenant fut l'absence de transactivation observée avec le variant Tat Oyi. Aucune transactivation n'a été observée non plus avec le dérivé Tat Bru possédant les cystéines carboxyméthylées. L'étude par dichroïsme circulaire (CD) en phase aqueuse de ces protéines synthétiques montre bien que la modification chimique de Tat Bru cmC a modifié de manière significative la structure de Tat Bru. Par contre Tat Oyi a une structure similaire aux autres comme l'atteste son spectre CD.

La souche VIH-1 Oyi a été identifiée chez un patient Gabonais (plus précisément une femme enceinte) en 1988 (Huet et al., 1989). Ce patient, bien que séropositif depuis plusieurs années, était en parfaite santé. Apparemment, le fait d'avoir une protéine Tat défectueuse dans cette souche VIH-1, a empêché l'évolution vers un SIDA chez ce patient et lui a conféré une immunité contre le virus du SIDA. En effet, les études épidémiologiques effectuées sur le terrain ont montré que les patients infectés par le VIH-1 Oyi étaient des non progresseurs à long terme.

La souche VIH Oyi appartient au sous-type B correspondant aux souches VIH-1 les plus répandues en Europe et en Amérique du Nord. La femme gabonaise chez laquelle la souche VIH Oyi a été identifiée appartenait à un groupe de 31 personnes localisées dans la province rurale du Haut Ogooué dans le sud est du Gabon. Ce groupe avait été identifié en 1986 lors d'une analyse séro-épidémiologique effectuée sur environ 2 000 personnes infectées dans l'ensemble du Gabon. Ce groupe du Haut Ogooué avait attiré l'attention car les personnes infectées étaient en bonne santé et présentaient également un profil Western Blot tout à fait atypique. En effet, il n'était pas possible de détecter, chez ces personnes, la présence d'anticorps anti *gp* 120, *gp* 160 et *gp* 41 alors que les anticorps anti *gag* et *pol* étaient identifiés (Huet et al., 1989).

Un groupe de 750 femmes enceintes, originaires de cette même province du Haut Ogooué, fut ensuite étudié et révéla que 25 d'entre elles (soit 3,3 %) étaient VIH-positives en test ELISA. Parmi ces 25 femmes, 23 avaient un profil Western Blot atypique caractéristique de la région (voir ci-dessus). Une dizaine de femmes a été suivie pendant 2 ans dont celle possédant la souche VIH-1 Oyi. Pendant cette période, le profil sérologique atypique resta constant excluant la possibilité d'une infection récente qui n'aurait pas laissé le temps à la formation d'anticorps anti *gp* 120. La présence d'une infection au VIH-2 fut exclue car il n'y avait pas de réaction à *gag* VIH-2 et uniquement 2 cas de contamination VIH-2 avaient été reportés au Gabon, localisés dans une région littorale. Tous les patients suivis restèrent en bonne santé avec aucune perte de poids ou maladie opportuniste pendant les 2 années de l'étude (Huet et al., 1989).

La femme identifiée Oyi était d'origine rurale, en bonne santé et séronégative pour HTLV-1 et VIH-2. La co-culture de ses lymphocytes avec des PPMC révéla une activité RT seulement après 15 jours, ce qui est très inhabituel. Le virus avait également une activité cytopathique presque indécelable. Bien que du surnageant de culture permettait l'infection de PBMC, pratiquement aucune réplication ne fut possible sur des lignées lymphocytaires normales comme les H-9 et CEM ou encore des monocytes U-937. Pour valider cette analyse, des résultats similaires furent observés avec 17 autres patients de la même région confirmant l'absence d'anticorps anti *gp* 120 dans tous les cas. Il est intéressant de constater que le sérum d'un donneur VBH-1 normal est capable de reconnaître la *gp* 120 de VIH-1 Oyi (Huet et al., 1989). Le virus VIH-1 Oyi a donc été cloné, le séquençage ne révéla aucune anomalie à l'exception du gène *Tat* (Huet et al., 1989).

La mutation de Cys 22 en Ser semble être la raison de la perte de la transactivation et la réversion de cette mutation permet de restaurer l'activité Tat.

Par ailleurs, il a été constaté qu'en l'absence de Tat, la reproduction du virus est possible mais à un niveau très faible. Il existe donc un rapport étroit entre la virulence du VIH et l'efficacité de transactivation de Tat. Cette étude épidémiologique montre encore une fois le lien étroit qui existe entre la mortalité de cette maladie et le taux de réplication du VIH. Tat semble donc permettre au VIH d'atteindre le taux de réplication qui transforme cette infection virale en maladie mortelle.

De plus, cette étude de ces patients atypiques de la région du Haut Ogooué a révélé la protection que semblerait procurer les souches VIH défectueuses vis-à-vis des souches VIH normales. En effet, des analyses par PCR de lymphocytes fraîchement prélevés chez plusieurs patients atypiques auraient révélé la présence de souches VIH normales. Le mécanisme de protection ne fait pas intervenir des anticorps anti *gp* 120, lesquels sont absents chez ces patients. L'action des lymphocytes T cytotoxiques pourrait être le mécanisme qui a permis l'éradication du virus.

Une étude épidémiologique relativement récente faite au Gabon (Delaporte et al., 1996) indique un faible pourcentage de personnes infectées par le VIH (2 à 3 %) par rapport à d'autres pays africains. Le sous-type Oyi semble avoir complètement disparu de la population et la femme gabonaise chez qui la souche VIH-1 Oyi avait été prélevée était en parfaite santé en 1995 et avait donné naissance à 3 enfants, tous séronégatifs. Le virus VIH-1 Oyi n'était plus détectable chez elle.

La protéine Tat du variant VIH-1 Oyi semble donc être le meilleur candidat pour entrer dans la composition d'un vaccin anti-VIH-1. Cependant, une Tat inactive n'est pas nécessairement immunogène. Or tout l'intérêt d'un vaccin est de générer la production d'anticorps qui plus est, dans le cas présent, des anticorps actifs à l'encontre d'autres variants de Tat. De façon surprenante, les Inventeurs ont pu faire cette démonstration. Le vaccin conforme à l'invention comprend donc, selon un mode de réalisation particulièrement intéressant, la Tat, en tout ou en partie, du variant VIH-1 Oyi.

Les avantages d'un vaccin anti-Tat peuvent même être envisagés à plusieurs niveaux. Chez les patients asymptomatiques, il serait possible de retarder la progression vers le SIDA en limitant l'immuno-déficience. Un tel vaccin pourrait permettre au patient de conserver une immunité efficace contre le virus, comme cela semble être le cas au début de l'infection. En particulier, la restauration de l'activité des lymphocytes T cytotoxiques (LTC) et des macrophages, qui ne sont pas infectés par le VIH mais dont l'activité est inhibée par Tat, pourrait avoir pour conséquence de permettre aux patients de devenir non progresseurs.

Chez les patients ayant un SIDA déclaré, un vaccin anti Tat pourrait limiter l'incidence de certaines pathologies tel que le sarcome de Kaposi ou des syndromes neurologiques qui semblent être liés à une action directe de la protéine Tat suite à leur sécrétion par les cellules infectées par le VIH.

L'intérêt du variant VIH-1 Oyi dans le cadre de la présente invention est d'ailleurs confirmé par les expériences de Western Blot (photographies de gel non montrées dans la présente demande) et le test Elisa réalisés par les Inventeurs afin de déterminer s'il existait des reconnaissances croisées entre les différents variants de Tat.

En effet, dans ces deux types d'expérimentation, trois sérums différents anti-Tat ont été testés à l'encontre de sept variants Tat.

Les Inventeurs ont immunisé des lapins avec Tat Oyi, Tat Bru cmC et Tat Eli selon un protocole d'immunisation classique en présence d'adjuvant de Freund. Les serums polyclonaux obtenus après 53 jours ont été analysés en western blot et en test Elisa. Les titres observés sont respectivement de 700, 11 et 660 pM pour les anticorps anti Oyi, Bru cmC et Eli.

Dans des conditions dénaturantes, les expériences de werstern blot montrent que le sérum anti-Tat Oyi et le sérum anti-Tat Eli sont incapables de montrer une immogénicité croisée avec tous les variants comme c'est le cas avec le sérum anti-Tat Bru cmC.

Tat Bru cmC est un variant dont les cystéines sont carboxyméthylées. Cette modification chimique des cystéines entraîne non seulement la perte de l'activité de transactivation (Péloponèse et al., 1999) mais également la perte de la structure 3D de ce variant qui se transforme en pelote statistique (résultat non publié). Il n'est donc pas surprenant de constater que le sérum anti-Tat Bru cmC est capable de reconnaître tous les variants lorsqu'ils sont dénaturés.

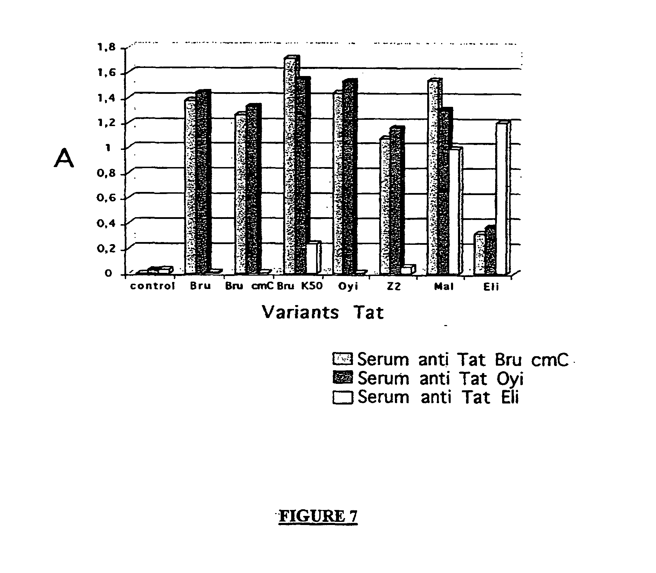

Dans des conditions non dénaturantes, le test Elisa (Figure 7) montre que le sérum anti-Tat Oyi est capable de reconnaître tous les variants comme le sérum anti-Tat Bru cmC. Par contre le sérum anti-Tat Eli est toujours incapable de reconnaître tous les variants. Il semble donc qu'il existe des épitopes 3D conservés chez de nombreux variants Tat. Toutefois, seul le caractère immunogénique particulier de Tat Oyi permet de générer ces anticorps en grande quantité. Il y a donc une forte probabilité pour que la présence de Tat chez les patients ait été sous-estimée jusqu'à présent. La dilution des trois sérums est de 1/1000. Dans la même expérience mais sans dilution, le sérum anti-Tat Eli peut également reconnaître tous les autres variants Tat. Il est intéressant de constater que l'acétylation de la lysine 50 de Tat Bru (Tat Bru K50) permet une reconnaissance par le sérum anti-Tat Eli à faible concentration.

Ces expériences effectuées avec ces trois sérums polyclonaux de lapin confirment donc l'intérêt de Tat Oyi comme vaccin. De plus, le sérum anti-Tat Oyi est capable d'inhiber la transactivation sur cellules HeLa avec Tat Bru ou Tat Eli. Le sérum anti-Tat Eli utilisé comme témoin montre que la production d'anticorps à partir d'un variant même actif ne garantit pas la reconnaissance croisée de tous les variants Tat comme cela est observé avec Tat Oyi. Tat Oyi, qui est un variant long (101 résidus), permet une meilleure reconnaissance des variants Tat par rapport à Tat Bru cmC qui est un variant court (86 résidus). Les souches HIV-1 avec des variants Tat courts ont pratiquement disparu et semble avoir été un artéfact de laboratoire (Jeang et al., 1999). De plus, le sérum anti-Tat Oyi présente une grande spécificité puisqu'il ne reconnaît plus les formes dénaturées (ou épitopes linéaires) des variants Tat. A l'inverse, le risque n'est pas négligeable pour le sérum anti-Tat Bru cmC de générer des anticorps auto-immuns chez l'être humain, du fait de la grande diversité structurale de Tat Bru cmC. Les Inventeurs ont observé en effet que dans le sérum anti-Tat Bru cmC il existait des anticorps capables de reconnaître l'albumine humaine (données non montrées).

La préservation de la structure tridimensionnelle d'au moins un épitope de Tat est donc essentielle à la reconnaissance d'autres variants de Tat par les anticorps anti-Tat 3D.

Par ailleurs, les Inventeurs ont également montré que les anticorps anti-Tat Oyi étaient capables d'inhiber la transactivation des protéines Tat Eli.

La présente invention a donc également pour objet un procédé de préparation d'anticorps anti-Tat monoclonaux ou polyclonaux aptes à reconnaître plusieurs variants de Tat. En effet, il est à souligner que ces anticorps peuvent donc être utilisés afin de détecter, chez les individus susceptibles d'avoir été infectés par le VIH, la présence de la protéine Tat dans leur sang quel qu'en soit le variant.

La présente invention concerne donc un procédé de préparation d'anticorps polyclonaux anti-Tat aptes à reconnaître plusieurs variants de Tat, comprenant :
- l'immunisation d'un animal excepté l'homme au moyen d'une protéine Tat synthétisée chimiquement associée à un adjuvant de la réponse immunitaire,
- la purification des anticorps spécifiques contenus dans le sérum des animaux immunisés,
la protéine Tat utilisée dans l'étape d'immunisation correspondant au variant Oyi.

L'immunisation de l'animal peut être effectuée par tout mode d'introduction de la protéine Tat ou partie de cette protéine Tat dans cet animal telle que : injection, inhalation, administration orale, sous cutanée intradermique, intrapéritonéale, intraveineuse ou intramusculaire.

L'immunisation de l'animal peut également être effectuée au moyen d'une expression *in vivo* et *in situ* d'un vecteur portant non seulement la séquence de la protéine Tat en tout ou partie mais également tout le système nécessaire à l'expression de la protéine Tat selon des techniques bien connues de l'homme du métier.

La purification des anticorps spécifiques contenus dans le sérum des animaux immunisés peut par exemple être effectuée sur une colonne d'affinité sur laquelle la protéine ou partie de la protéine ayant servi d'antigène a été préalablement fixée.

La position de sept résidus cystéine (région 22-37) est très conservée parmi tous les variants Tat et l'importance de 6 de ces résidus cystéine pour la transactivation est largement décrite dans la littérature (Jeang, 1996)

Par conséquent, il est tout à fait envisageable de muter un variant Tat sur au moins l'une de ces cystéines afin de rendre ledit variant incapable de transactiver tout en ayant les propriétés recherchées dans le cadre de la présente invention. Cette mutation peut par exemple consister en une substitution de la cystéine en un autre acide aminé tel qu'une sérine ou une alanine.

Les Inventeurs, par la détermination de la structure 3D de Tat Bru par RMN 2D hétéronucléaire, ont par ailleurs identifié une région cible qui devrait être conservée chez les variants Tat. Cette cible est constituée en partie par la région N-terminale et la région basique de la séquence de Tat.

La structure 3D du variant Tat Bru montre que la région riche en cystéines est proche de l'espace tridimensionnel de la région N-terminale et de la région basique. Ceci indique que la carboxyméthylation des cystéines modifie probablement la structure de cette cible et confirme le fait que la Tat ainsi modifiée ne peut être considérée comme un candidat potentiel pour la préparation d'un vaccin anti-VIH.

Par ailleurs, les inventeurs ont réalisé la synthèse chimique en phase solide des différentes protéines Tat. Au stade actuel des connaissances dans le domaine, les avantages présentés par ce mode de synthèse résident dans les coûts de production moindres, les meilleurs rendements par rapport à la Tat produite par biologie moléculaire et l'absence de contaminations.

Ainsi, de manière avantageuse, la Tat mise en oeuvre dans le vaccin conforme à l'invention est préparée par synthèse chimique et plus particulièrement par synthèse chimique en phase solide, telle qu'une synthèse de type FMOC et de préférence fastFMOC. Un procédé de synthèse mettant en oeuvre des groupes protecteurs de ce type est également un objet de la présente invention.

Cependant, la Tat mise en oeuvre dans le vaccin conforme à l'invention peut également être synthétisée d'une toute autre manière, par exemple au moyen de techniques recombinantes bien connues de l'homme du métier. Ces systèmes de clonage et d'expression utilisés dans le cadre de ces techniques peuvent être issus de microorganismes tels que *Escherichia coli,* Bacillus, Streptomyces et Saccharomyces mais également à partir de cellules de levure, d'insectes et de mammifères. Les systèmes d'expression à partir de Baculovirus sont également envisageables. Bien évidemment, les vecteurs portant la séquence de la protéine Tat en tout ou partie comprendront tout le système d'expression nécessaire pour ce faire, selon des techniques bien connues de l'homme du métier.

De plus, afin d'éviter tout risque potentiel de toxicité du fait du passage de la protéine Tat, en particulier Tat Oyi, dans les membranes, il conviendrait de modifier la séquence Arg-Gly-Asp présente en C-terminal de la séquence de tous les variants Tat. Cette séquence est en effet essentielle pour le passage membranaire car elle reconnaît un récepteur membranaire à la surface des cellules (Jeang, 1996). Plus particulièrement, cette modification pourrait consister en la substitution de cette séquence par la séquence Lys-Ala-Glu ce qui n'aurait pas d'influence sur la structure, ou éventuellement par la séquence Ala-Ala-Ala.

Comme indiqué plus haut, il est très probable que l'on ait sous-estimé jusqu'à présent le nombre de personnes infectées par le VIH faute d'avoir pu déceler chez elles, par exemple dans leur sang, la présence de la protéine Tat. L'intérêt de la présente invention est de fournir les moyens de déceler le plus grand nombre possible de variants Tat au moyen d'un nombre restreint d'anticorps.

L'invention a donc également pour objet un procédé de mise en évidence de la présence de la protéine Tat ou d'une partie de la protéine Tat dans un échantillon biologique, comprenant :
- la mise en contact dudit échantillon avec des anticorps anti-Tat capables de donner lieu à des complexes antigène-anticorps avec plusieurs variants de Tat,
- la détermination de la présence des complexes antigène-anticorps.

Par « anticorps anti-Tat », on entend non seulement des anticorps entiers mais également des fragments d'anticorps ou anticorps chimériques capables de remplir la fonction recherchée dans le cadre de la présente invention, à savoir reconnaître tout ou partie de la protéine Tat.

La détermination de la présence des complexes antigène-anticorps se fait selon des méthodes bien connues de l'homme du métier. En particulier, les réactifs permettant la détection de ces complexes peuvent porter un marqueur ou être susceptibles d'être reconnus à leur tour par un réactif marqué, plus particulièrement dans le cas où l'anticorps utilisé n'est par marqué.

La mise en contact de l'échantillon biologique dans le procédé conforme à l'invention avec des anticorps anti-Tat peut également être effectuée avec un sérum anti-Tat.

Par « échantillon biologique » on entend tout échantillon liquide, cellulaire ou tissulaire prélevé chez un patient et susceptible de contenir l'antigène apte à réaliser un complexe antigène-anticorps en présence du ou des anticorps approprié(s). De préférence, cet échantillon biologique est du sang.

Enfin, la présente invention a également pour objet une trousse de diagnostic de l'infection par le VIH par la détermination de la présence de la protéine Tat ou d'une partie de la protéine Tat dans un échantillon biologique, comprenant :
- des réactifs pour la constitution d'un milieu propice à la réaction immunologique,
- des réactifs permettant la détection des complexes antigène-anticorps produit par la réaction immunologique,
- éventuellement, un échantillon biologique de référence (témoin négatif) dépourvu d'antigène,
- éventuellement, un échantillon biologique de référence (témoin positif) contenant une quantité prédéterminée d'antigènes.

Selon un mode de réalisation avantageux de la présente invention, les réactifs pour la constitution du milieu propice à la réaction immunologique comprennent des anticorps anti-Tat capables de donner lieu à des complexes antigènes-anticorps avec plusieurs variants Tat et de préférence comprennent des anticorps anti-Tat Oyi, anti-Tat Bru cmM ou une combinaison des deux.

### FIGURES

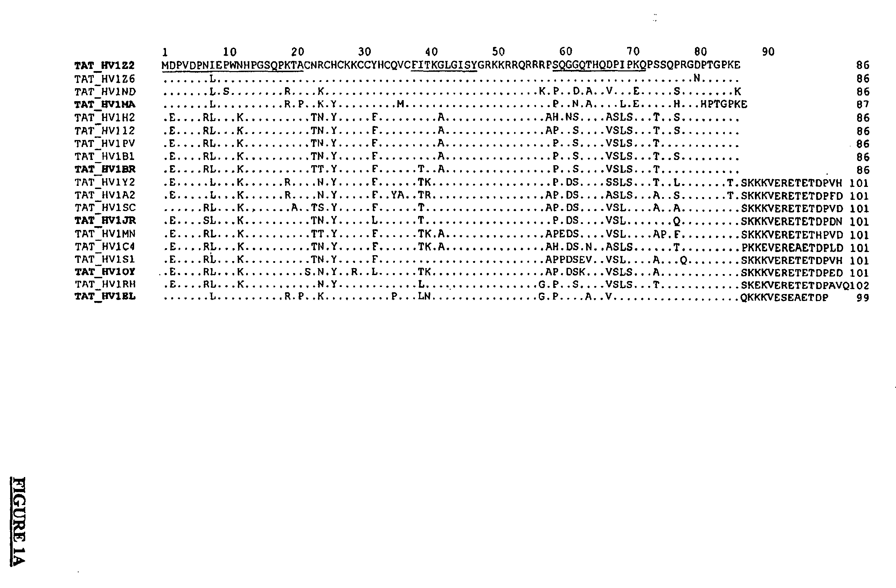

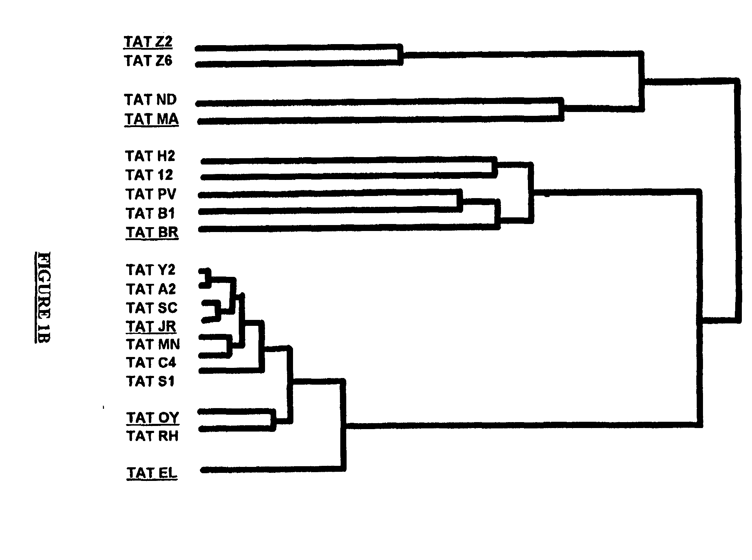

**Figure 1** : Comparaison des différentes protéines Tat.
   Les protéines Tat ont été réparties en 6 groupes structuraux qui sont mentionnés en gras sur la Figure 1A et soulignés sur la Figure 1B.
Figure 1A : Séquences en acides aminés des protéines Tat.
   TatZ2 provient d'un isolat de VIH-1 proche des souches ancestrales du virus (Zhu et al., 1998). Tat Mal et Tat Eli proviennent d'une souche VIH-1 isolée pendant les années 80 en Afrique Centrale à l'occasion d'infections hétérosexuelles à VIH (Alizon et al., 1986). Tat Br comprend la séquence la plus utilisée en laboratoire et provient d'une souche VIH-1 isolée en France (Barre-Sinoussi et al., 1983), tandis que Tat Jr provient d'un isolat VIH-1 américain (O'Brien et al., 1990). Tat Oyi est étroitement apparenté à Tat Jr et Tat Bru mais provient d'une souche VIH identifiée chez un patient sein au Gabon (Huet et al., 1989).
Figure 1B : Classification des protéines Tat en fonction de leur taille et des mutations qu'elles comprennent pour le programme MULTALIN (Corpet, 1989).

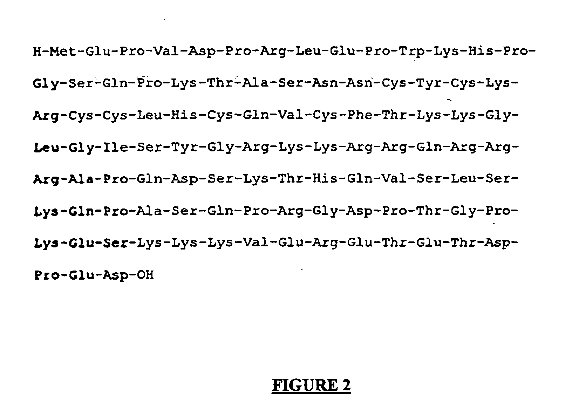

**Figure 2** : Séquence de la protéine Tat du variant VIH-Oyi (décrite par Huet et al, 1989)
**Figure 3** : Purification des 6 variants Tat par chromatographie liquide haute performance en phase reverse avec colonne greffée C8 à 280 nm (voir Matériels et Méthodes pour la procédure expérimentale).
   Pour chaque cadre, le tracé inférieur représente le résultat de la synthèse peptidique avant purification, tandis que le tracé supérieur représente le résultat de la synthèse après la dernière étape de purification. Tat Bru (A), Tat Jr (B), Tat Z2 (C), Tat Oyi (D), Tat Mal (E) et Tat Eli (F) ont été clivées à partir de la résine avec TFA. Après précipitation avec de l'éther de butyle méthyle, les protéines ont été dissoutes dans du Tampon TFA à 0,1 % (tracé inférieur dans chaque cadre). Pour chaque protéine, la purification a été effectuée avec 2 migrations HPLC sur colonnes Hybar Merk C8 (4,5 x 125 mm, débit 0,8 ml/min) semi-préparatives successives et ensuite les fractions pures ont été analysées (tracé supérieur de chaque cadre) et identifiées par spectrométrie de masse et par une analyse des acides aminés (données non montrées). Dans chaque cas, il s'avère que le pic principal HPLC représente la séquence complète. Les pics entre 10 et 15 min représentent des dérivés de 50 résidus tandis que les pics proches de la fraction principale sont des dérivés avec 1 à 15 délétion(s) à partir de l'extrémité N-terminale. Les fractions hautement hydrophobes sont des dérivés avec un poids moléculaire élevé probablement dû aux chaînes latérales incomplètement déprotégées.

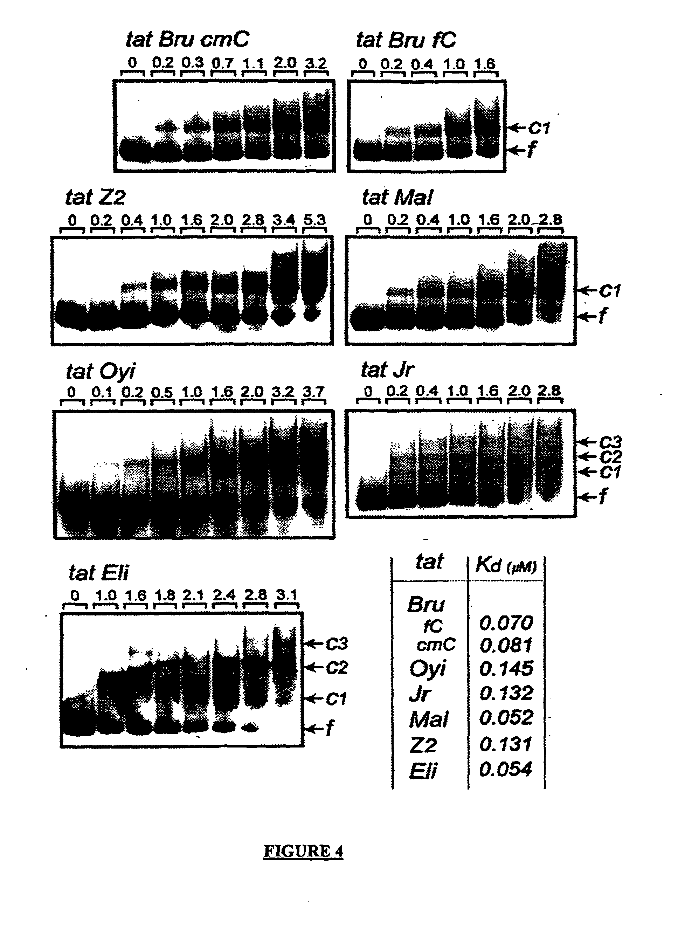

**Figure 4** : Mesure de la constance d'affinité des Tat synthétiques pour la cible nucléotidique TAR par électrophorèse (voir les tests de déplacement de mobilité électrophorétique dans Matériels et Méthodes.
   La concentration protéique (ng/µl) est indiquée au sommet de chaque bande de gel. L'ARN libre ou complexé est identifié par *f* (pour free/libre) et *c* respectivement. La préparation du même ARN est utilisée pour la titration avec les 6 protéines. Un dérivé Tat Bru avec des cystéines carboxyméthylées (Bru CmC) a également été testé. Les constantes de dissociation à l'équilibre (Kd) ont été mesurées directement à partir de tests de déplacement de mobilité électrophorétique. Les valeurs de Kd varient approximativement de 50 nM pour Tat Eli et Tat Mal à environ 140 nM pour Tat Oyi et Tat Jr. De plus, les inventeurs ont noté qu'au-delà de la variation des valeurs de Kd, les profils de liaison avec les diverses protéines sont quelques peu différents. Par exemple, des concentrations faibles de Tat Bru produisent un complexe simple bien résolu et ce n'est qu'avec des concentrations plutôt supérieures (> 4,5 ng/µl) que des agrégats se forment et ne peuvent pas pénétrer dans les gels d'acrylamide. Au contraire, des complexes multimères sont aisément identifiés avec Tat Eli même à de faibles concentrations. On peut voir jusqu'à 3 bandes retardées avec Tat Eli, dans une moindre mesure avec Tat Jr. De tels effets ne sont pas observés avec des protéines plus courtes telles que Tat Bru et Tat Mal par exemple, ainsi qu'avec Tat Oyi qui est une protéine plus longue.

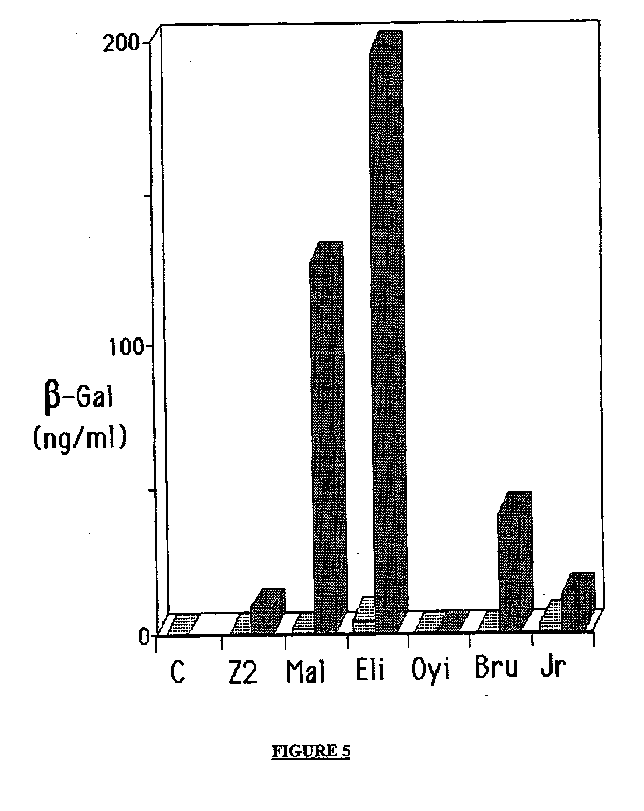

**Figure 5** : Test de transactivation des protéines Tat synthétiques sur cellule HeLa. Ces cellules sont transfectées par le LTR du VIH-1 auquel est associé un gène rapporteur LacZ. Le taux de protéine β-gal produit est proportionnel à la capacité de transactivation des différentes Tat synthétiques. Le LTR contient les séquences d'ADN en aval et en amont requises pour la transcription du VIH et TAR est présent au début de l'ARNm (Clavel et Charneau, 1994). Les cofacteurs cellulaires requis pour la transactivation du VIH sont présents dans les cellules HeLa. Sans Tat, il y a une expression basale de β-gal qui est indiquée comme contrôle (C). L'histogramme suivant montre la transactivation observée avec les différents variants de Tat utilisant 2 concentrations : 1 µM (boîte gris clair) et 5 µM (boîte gris foncé). Dans chaque case, Tat a été additionnée au tampon cellulaire et donc une expression de β-gal supérieure au contrôle signifie que la Tat synthétique est capable de traverser les membranes nucléaires et cytoplasmiques, de se lier à TAR et d'interagir avec des cofacteurs cellulaires. Seul Tat Bru cmC (donnée non montrée) et Tat Oyi sont déficiemes dans cette expérimentation alors qu'elles se lient à TAR (Figure 1). Tat Mal et Tat Eli montrent un niveau de transactivation 3 à 4 fois supérieur à celui de Tat Bru. Tat Z2, le plus proche des protéines Tat ancestrales, présente un faible niveau de transactivation et l'évolution pourrait favoriser les isolats de VIH-1 avec une Tat plus efficace. Des résultats similaires ont été obtenus avec d'autres tests de transfection utilisant la luciférase comme gène reporter et Tat Mal et Tat Eli à 1 µM transactivaient bien le LTR à un niveau comparable à celui obtenu avec une Tat-pCMV transfectée (donnée non montrée). La gamme de concentrations utilisées dans les différentes expérimentations s'étend de 0,1 µM à 10 µM (donnée non montrée). A 0,1 µM, seule Tat Eli montre un niveau de transactivation significativement supérieur au niveau de base. A 10 µM, les 6 Tat montrent des niveaux de transactivation tellement élevés qu'il est impossible d'observer des différences entre elles en raison de la saturation.

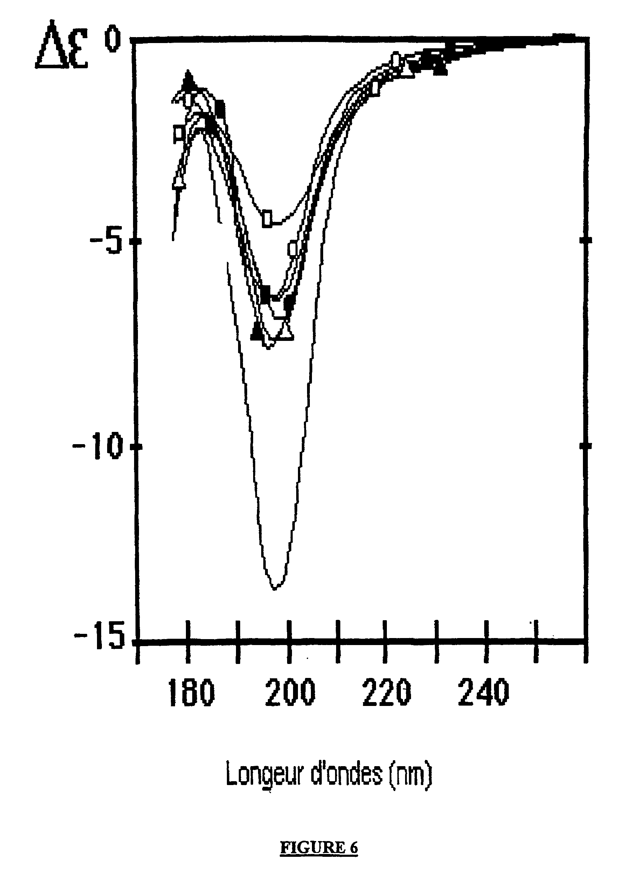

**Figure 6** : Spectres dichroïques des variants Tat Z2 (triangle blanc), Tat Oyi (triangle noir), Tat Bru (cercle blanc), Tat Bru cmC (pas de marque), Tat Jr (cercle noir), Tat Mal (carré blanc) et Tat Eli (carré noir).
   Les spectres sont mesurés en tampon phosphate à 20 mM pH 4,5, ils sont enregistrés de 260 à 178 nm avec un trajet optique de 50 µm. Les différences observées dans les spectres CD révèlent une hétérogénéité structurale entre les variants Tat quelle que soit leur taille. Il n'est pas possible de rassembler les spectres CD dans 2 catégories constituées des Tat courtes (marques blanches) et des Tat longues (marques noires). Les spectres CD sont caractérisés par une bande négative proche de 200 nm typique des structures non organisées. La magnitude intense de la bande de 200 nm observée avec Tat Bru cmC montre que la modification des cystéines a entraîné des changements conformationnels majeurs par rapport aux autres Tat.
**Figure 7** : Tests Elisa effectués avec trois dilutions de sérums polyclonaux de lapin (anti-Tat Bru cmC, anti-Tat Oyi et anti-Tat Eli) à l'égard de 7 variants de la protéine Tat dans les conditions dénaturantes.

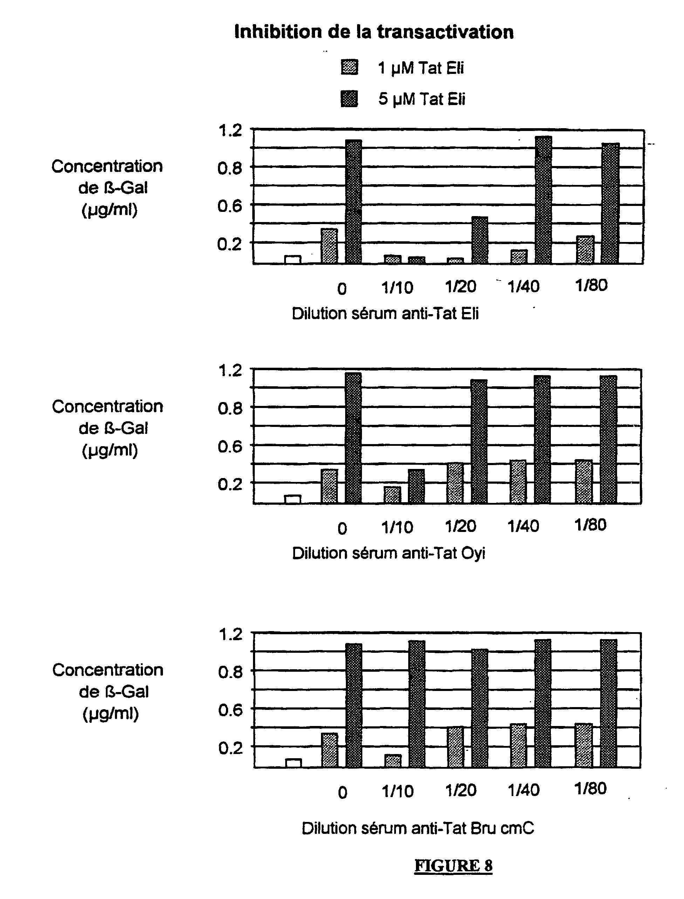

**Figure 8** : Inhibition de la transactivation par Tat Eli avec les sérums anti-Tat Eli, anti-Tat Oyi et Tat Bru cmC.

La présente invention ne se limite pas à la description ci-dessus. Elle sera mieux comprise à la lumière des exemples qui ne sont mentionnés qu'à titre illustratif.

### EXEMPLES

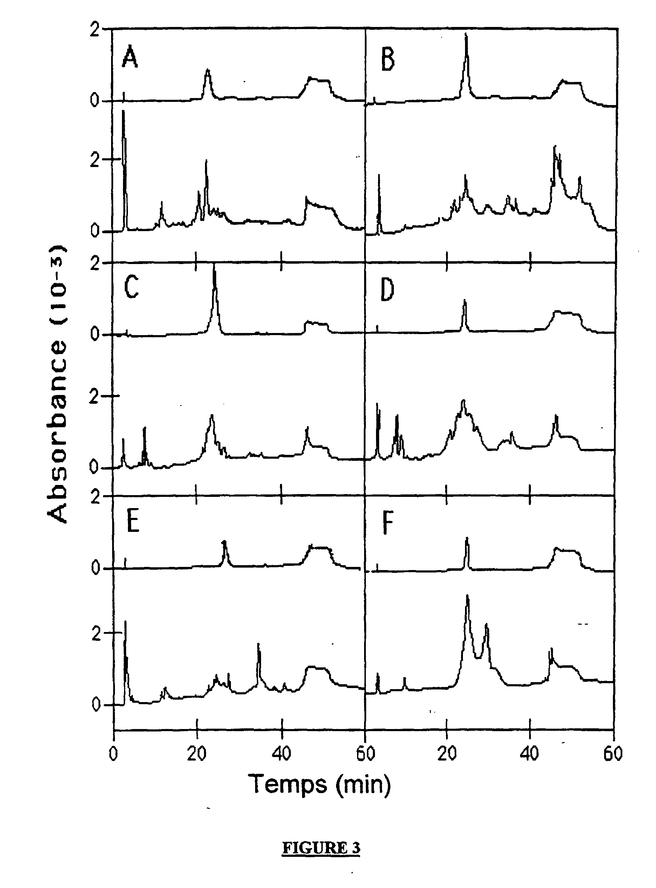

### 1) MATERIELS ET METHODES CORRESPONDANT AUX FIGURES 3 A 6

### Synthèse protéique, purification et caractérisation

Des peptides ont été assemblés selon la méthode de Barany et Merrifield (1980) sur une résine préchargée de divinylbenzène de 4-hydroxyméthyl-phénoxyméthyl-copolystyrène à 1 % (HMP) (0,5-0,65 mmol) (Perkin Elmer, Applied Biosystem Inc., Forster City, CA) sur un synthétiseur automatisé (ABI 433A, Perkin Elmer, Applied Biosystem Inc., Forster City, CA). De façon à éviter l'obtention de dérivés présentant des délétions, les extrémités N-terminales sans Fmoc ont été protégées par un groupement acétyl après traitement par un mélange comprenant 4,75 % d'anhydre acétique (Merck), 6,25 % de DIEA à 2,0 M, 1,5 % de 1-hydroxybenzotriazol IM (HOBt) (Perkin Elmer, Applied Biosystem Inc., Warrington, GB) et 87,5 % de N-méthylpirrolidone (Perkin Elmer, Forster City, CA). Chaque étape de déprotection a été contrôlée avec un dispositif de conductivité. Les peptides ont été déprotégés et retirés de la résine avec de l'acide trifluoroacétique (TFA) complémenté avec 10 % de méthyl-phényl-sulphide (Merck) et 5 % d'éthane dithiol (Merck). La purification a été réalisée avec un appareil de chromatographie liquide à haute performance de Beckman (HPLC) et une colonne en phase reverse C8 de Merck (10 x 250 mm). Le Tampon A était constitué d'eau avec 0,1 % de TFA et le Tampon B était constitué d'acétonitrile avec 0,1 % de TFA. Le gradient était constitué de Tampon B de 20 à 40 % en 40 min et un débit de 2 ml/min. Une spectrométrie de masse par électrovaporisation a été réalisée avec un PE-SCIEX API 150ex simple quad de Perkin Elmer. Des analyses d'acides aminés ont été réalisées sur un analyseur de Beckman, modèle 6300.

### Tests de déplacement de mobilité électrophorétique

Les 59 nucléotides de l'ARN de TAR contenant le renflement pyrimidine UUU essentiel a été préparé *in vitro* par transcription avec l'ARN polymérase T3. Les mélanges de réaction de liaison (20 µl) contenaient 0,2 nmole d'ARN de TAR radiomarqué, 0-100 ng de Tat dans un Tampon TK (50 mM de Tris pH 7,4, 20 mM de KCl, 0, 1 % de Triton X-100). Les complexes ont été séparés de l'ARN non lié par électrophorèse sur des gels dénaturants de polyacrylamide à 8 % contenant 0,1 % de Triton X-100. Le gel a été pré-migré pendant 30 min avant le chargement de l'échantillon (25 µl). L'électrophorèse s'est poursuivie pendant 90 min à environ 200 V. Les quantités relatives d'ARN libre et/ou lié ont été déterminées par imagerie au phosphore.

### Dichroïsme circulaire (CD)

Les spectres de dichroïsme circulaire ont été mesurés avec un trajet optique de 50 µm de 260 à 178 nm sur un spectrophotomètre CD UV de Jobin-Yvon (Long-Jumeau, FRANCE) (Mark VI). L'instrument a été calibré avec de l'acide (+)-10-camphorsulfonique. Un rapport de 2,1 a été trouvé entre la bande CD positive à 290,5 nm et la bande négative à 192,5 nm. Les données ont été collectées à 0,5 nm d'intervalle avec un taux de balayage de 1 nm par minute. Les spectres CD ont été reportés sous forme de Δε par amide. Les échantillons ont été préparés dans un tampon phosphate à 20 mM (pH 4,5). Les concentrations protéiques étaient comprises dans une gamme de 0,5 à 1 mg/ml.

### Transactivation avec des cellules transfectées par LTR de HIV

La transactivation fonctionnelle par une Tat synthétique a été déterminée en utilisant des cellules P4. Ces cellules CD4-HeLa portent le gène lacZ bactérien sous le contrôle de LTR de VIH et l'accumulation cytoplasmique de β-galactosidase est strictement dépendante de la présence de Tat. 80 % des cellules confluantes étalées sur une plaque à 12 puits ont été incubées pendant 24 h à 37°C, en présence de CO₂ à 5 %, avec la protéine Tat comprise dans un milieu DMEM supplémenté avec 0,1 % de BSA. Suite à cette période d'incubation, les cellules ont été lavées avec une solution saline tamponnée au phosphate et les protéines ont été extraites et analysées pour la β-galactosidase avec un test d'immunoabsorption du commerce mettant en oeuvre une enzyme liée à un antigène (ELISA pour β-galactosidase, Boehringer Mannheim, FRANCE), selon les instructions du fabricant. Les valeurs ont été normalisées au moyen de la concentration des protéines totales des différents lysats cellulaires comme déterminé par la méthode de Bradford.

### Inhibition de la transactivation

Les expérimentations ci-dessus de transactivation avec des cellules transfectées par LTR du VIH ont été reproduites mais cette fois avec ajout au milieu de culture soit d'un sérum anti-Tat Eli, soit d'un sérum anti Tat Oyi., soit d'un sérum anti Tat Bru. La protéine Tat provenait quant à elle des variants Eli.

Les inventeurs ont observés une inactivation de la transactivation par les anticorps anti-Tat Oyi de la protéine Tat Eli en comparaison notamment avec le milieu contrôle qui ne comprenait pas d'anticorps.

### 2) MATERIELS ET METHODES CORRESPONDANT A LA FIGURE 7

Les conditions d'immunisations sont une injection intradermique de 100 µg de protéine Tat purifiée dans 0,5 ml de Tampon phosphate pH 4,5 100 mM plus 0,5 ml d'adjuvant complet de Freund (Jour 0). Un premier rappel est effectué à J21 dans les mêmes conditions mais avec de l'adjuvant incomplet de Freund (0,5 ml). Un deuxième rappel est effectué à J42 identique à J41. Le sang est prélevé à J53 dans des tubes FST et le sérum est obtenu après centrifugation à 2000 tours/min. La dilution des sérums est 1/1000.

**Elisa :** Le test se fait sur une plaque Maxisorp U96 (Polylabo). Les protéines en tampon phosphate pH 4.5 sont incubées sur la plaque toute la nuit à 4°C. Après saturation en tampon MPBS (NaCl 8 g/L, KCl 0,2 g/L, Na2HPO4 1,44 g/L, KH2PO4 0,24 g/L, lait écrémé 5 % ajusté à pH 7,4 avec HCI), les protéines sont incubées avec l'anticorps primaire de lapin pendant une heure. Ensuite incubation avec un anticorps de chèvre couplé à la péroxydase spécifique du fragment Fc de lapin (Cappel) pendant une heure. La révélation se fait en présence d'H2O2, de 100 mM acide citrique, 50 mM NaOH et 0,2 mg/ml ABTS (Boerhinger). La lecture de l'absorbance se fait à 405 nm.

Sans dilution le sérum anti-Tat Eli est capable de reconnaître tout les variants ou dérivés Tat.

### 3) MATERIELS ET METHODES DU WESTERN BLOT

**Western Blot :** Les protéines sont d'abord dénaturées en présence de 3 M Tris-HCI, pH 8,8, 5 % β-mercaptoéthanol, 2 % SDS, 10 % glycérol, et Bleu de bromophénol. Elles sont séparées par électrophorèse sur gel polyacrylamide 15 %. Les protéines sont ensuite transférées sur feuilles de nitrocellulose afin d'êtres révélées par immunodétection. Les sites aspécifiques sont bloqués par une solution PBS (NaCl 8 g/L, KCI 0,2 g/L, Na2HPO4 1,44 g/L, KH2PO4 0,24 g/L, ajusté à pH 7,4 avec HCI) contenant du lait écrémé 5 %. La feuille de nitrocellulose est mise à incuber avec l'anticorps primaire de lapin pendant une heure. Ensuite incubation avec un anticorps de chèvre couplé à la péroxydase spécifique du fragment Fc de lapin (Sigma). La révélation se fait en précense d'H2CO2, PBS et diaminobezamidine. (Photographie du gel non montrée dans la présente invention).

### 4) MATERIELS ET METHODES CORRESPONDANT A LA FIGURE 8

La transactivation est mesurée avec des cellules humaines HeLa transfectées avec le LTR du VIH-1 et un gène rapporteur de la protéine β-Galactosidase (Lac Z) selon le protocole décrit pour la figure 5.

Les sérums sont obtenus chez le lapin selon le protocole décrit pour la figure 7.

Dans un milieu de culture de cellules, on ajoute 100 µl de trois sérums anti-Tat, à savoir anti-Tat Eli, anti-Tat Oyi et anti-Tat Bru cmC dans des dilutions croissantes, puis 100 µl du variant Tat Eli à 1 ou 5 µM. L'accumulation cytoplasmique de β-Galactosidase dépend donc de la présence de Tat.

Tout d'abord, on constate, à dilution équivalente, que le sérum anti-Tat Eli est le plus efficace. Ceci est logique car le sérum contient des anticorps produits à l'encontre du même variant que celui dont on cherche à neutraliser l'activité de transactivation. Cependant, il est à souligner que Tat Oyi est capable de générer des anticorps neutralisant de façon significative l'activité de transactivation de Tat Eli. Cette inhibition est d'ailleurs supérieure à celle observée notamment pour la dilution 1/10 au moyen du sérum anti-Tat Bru cmC. Ceci confirme l'intérêt des anticorps anti-Tat Oyi à l'encontre d'autres variants de Tat.

### REFERENCES

Alizon, M., Wain-Hobson, S., montagnier, L., & Sonigo, P. (1986) *Cell* **46**,63-74

Barany, G. & Merrifield, R.B. (1980) in Gross, E., & Meinhofer, J. (Eds) *The peptide: Analysis, Synthesis, Biology. Academic Press, New York,* Vol. 2, pp 1-284

Barre-Sinoussi, F., Chermann, J.C., Rey, F., Nugeyre, M.T., Chamaret, S., Gruest, J., Dauguet, C., Axler-Blin, C., Vezinet-Brun, F., Rouzioux, C., **Rozenbaum**, W. & Montagnier, L. (1983) *Science* **220**, 868-871

**Clavel, F. & Charneau, P.** (1994) *J. Virol* **68,** 1179-1185

**Corpet, F. (1989)** Nucl. Acid. Res. 22, 10881-10890.

Delaporte E., Janssens W., Peeters M., Buve A., Dibanga G., Perret J.L., Ditsambou V., Mba J.R, Courbot M.C., Georges A., Bourgeois A., Samb B., Henzel D., Heyndrickx L., Fransen K., van der Groen G., Larouze B. (1996) *AIDS 8,* 903-910

Grégoire, C. & Loret, E.P. 1996. *J. Biol. Chem.* **271,** 22641-22646

Huet, T., Dazza, M.C., Brun-Vezinet, F., Roelants, G.E. & Wain-Hobson, S. 1989. *AIDS* **3**, 707-715

Jeang, K.T. (1996) in Los Alamos National Laboratory (Ed) *HIV-1 Tat: Structure & Function*. Human Retroviruses & AIDS compendium. III, pp 3-18

Jeang, K. T., Xiao, H., & Rich, E. A. (1999) *J. Biol. Chem.* 274, 28837-28840.

Le Buanec, H., Lachgar, A., Bizzini, B., Zagury, J. F., Rappaport, J., Santagostino, E., Muca-Perja, M. & Gringeri, A 1998. *Biomed. Pharmacother* **10,** 431-435

O'Brien, W.A, Koyanagi, Y., Namazie, A, Zhao, J.Q., Diagne, A, Idler, K., Zack, J.A. & Chen, I.S. (1990) *Nature* **348,** 69-73

Westendorp, M.O., Frank, R., Ochsenbauer, C., Stricker, K., Dhein, J., Walczak, H., Debatin, K.M. & Krammer, P.H. 1995. *Nature* **375,** 497-500

Zhu, T., Korber, B.T., Nahmias, A.J., Hooper, E., Sharp, P.M. & Ho, D.D. (1998) *Nature* **391,** 594-597

## Revendications

1. Vaccin anti-VIH-1 comprenant une protéine Tat de VIH-1 comprenant entre 99 et 106 acides aminés, capable de se fixer sur TAR et incapable de transactiver, en association avec un véhicule pharmaceutiquement acceptable et, le cas échéant, un ou plusieurs adjuvant(s) de l'immunité approprié(s).

2. Vaccin selon la revendication 1, **caractérisé par le fait qu'**il s'agit d'une Tat dont la séquence nucléotidique présente au moins une mutation par rapport à la séquence nucléotidique d'une Tat fonctionnelle.

3. Vaccin selon la revendication 2, **caractérisé par le fait que** la mutation porte sur au moins l'une des cystéine de la proteine Tat.

4. Vaccin selon les revendications 1 à 3, **caractérisé par le fait qu'**il s'agit de la Tat du variant VIH-1 Oyi.

5. Vaccin selon l'une des revendications 1 à 4, **caractérisé par le fait qu'**il s'agit d'une Tat synthétique chimique.

6. Vaccin selon la revendication 5, **caractérisé par le fait que** la Tat est synthétisée en phase solide.

7. Vaccin selon l'une des revendications 1 à 4, **caractérisé par le fait qu'**il s'agit d'une Tat recombinante.

8. Vaccin anti-VIH, **caractérisé par le fait qu'**il contient un vecteur d'expression comprenant une séquence nucléotidique codant pour une protéine Tat de VIH-1 telle que définie dans la revendication 1, ainsi que les éléments nécessaires à son expression.

9. Vaccin selon la revendication 4 **caractérisé par le fait qu'**il comprend au moins la région N-terminale et/ou la région basique de la séquence de Tat.

10. Vaccin selon l'une des revendications 1 à 9, **caractérisé par le fait que** la région Arg-Gly-Asp située à l'extrémité C-terminale de Tat est modifiée.

11. Procédé de préparation d'anticorps polyclonaux anti-Tat aptes à reconnaître plusieurs variants de Tat, comprenant :
- l'immunisation d'un animal, excepté l'homme, par injection d'une protéine Tat telle que définie dans la revendication 5 associée à un adjuvant de la réponse immunitaire,
- purification des anticorps spécifiques contenus dans le sérum des animaux immunisés.
**caractérisé en ce que** la protéine Tat utilisée dans l'étape d'immunisation correspond au variant Oyi.

12. Procédé de mise en évidence de la présence de la protéine Tat ou d'une partie de la protéine Tat dans un échantillon biologique, comprenant :
- la mise en contact dudit échantillon avec des anticorps anti-Tat obtenus par la mise en oeuvre du procédé selon la revendication 11,
- la détermination de la présence des complexes antigène-anticorps.

13. Trousse de diagnostic de l'infection par le VIH par la détermination de la présence de la protéine Tat ou d'une partie de la protéine Tat dans un échantillon biologique, comprenant :
- des réactifs pour la constitution d'un milieu propice à la réaction antigène-anticorps, comprenant des anticorps obtenus par la mise en oeuvre du procédé selon la revendication 11,
- des réactifs permettant la détection des complexes antigène-anticorps,
- éventuellement, un échantillon biologique de référence (témoin négatif) dépourvu d'antigène,
- éventuellement, un échantillon biologique de référence (témoin positif) contenant une quantité prédéterminée d'antigène.

14. Procédé de synthèse d'un variant Tat tel que défini dans l'une des revendications 1 à 4, 9 et 10, **caractérisé en ce qu'**il met en oeuvre des groupements protecteurs de type FMOC, de manière préférentielle de type fastFMOC.

## Patentansprüche

1. Anti-HIV-1-Impfstoff, welcher ein Tat-Protein von HIV-1, welches zwischen 99 und 160 Aminosäuren umfasst, in der Lage ist, an TAR zu binden, und nicht in der Lage ist, zu transaktivieren, in Kombination mit einem pharmazeutisch annehmbaren Träger und gegebenenfalls einem oder mehreren geeigneten Immunitätsadjuvans bzw. -adjuvantien umfasst.

2. Impfstoff nach Anspruch 1, **dadurch gekennzeichnet, dass** es sich um ein Tat handelt, dessen Nukleotidsequenz wenigstens eine Mutation verglichen mit der Nukleotidsequenz eines funktionalen Tats aufweist.

3. Impfstoff nach Anspruch 2, **dadurch gekennzeichnet, dass** die Mutation sich auf wenigstens eines der Cysteine des Tat-Proteins bezieht.

4. Impfstoff nach den Ansprüchen 1 bis 3, **dadurch gekennzeichnet, dass** es sich um das Tat der HIV-1-Variante Oyi handelt.

5. Impfstoff nach einem der Ansprüche 1 bis 4, **dadurch gekennzeichnet, dass** es sich um ein chemisch synthetisiertes Tat handelt.

6. Impfstoff nach Anspruch 5, **dadurch gekennzeichnet, dass** das Tat mittels einer festen Phase synthetisiert wird.

7. Impfstoff nach einem der Ansprüche 1 bis 4, **dadurch gekennzeichnet, dass** es sich um ein rekombinantes Tat handelt.

8. Anti-HIV-Impfstoff, **dadurch gekennzeichnet, dass** er einen Expressionsvektor enthält, welcher eine Nukleotidsequenz, welche ein Tat-Protein von HIV-1, wie in Anspruch 1 definiert, kodiert, sowie die Elemente, die für dessen Expression erforderlich sind, umfasst.

9. Impfstoff nach Anspruch 4, **dadurch gekennzeichnet, dass** er wenigstens die N-terminale und/oder die basische Region der Tat-Sequenz umfasst.

10. Impfstoff nach einem der Ansprüche 1 bis 9, **dadurch gekennzeichnet, dass** die an dem C-terminalen Ende von Tat befindliche Region Arg-Gly-Asp modifiziert ist.

11. Verfahren zur Herstellung von polyklonalen anti-Tat-Antikörpern, welche dazu geeignet sind, mehrere Varianten von Tat zu erkennen, umfassend:
- die Immunisierung eines Tiers, mit Ausnahme des Menschen, durch Injektion eines Tat-Proteins, wie in Anspruch 5 definiert, in Kombination mit einem Adjuvans für die Immunantwort,
- Reinigung der spezifischen Antikörper, die in dem Serum der immunisierten Tiere enthalten sind,
**dadurch gekennzeichnet, dass** das in dem Immunisierungsschritt eingesetzte Tat-Protein der Variante Oyi entspricht.

12. Verfahren zum Nachweis des Vorhandenseins des Tat-Proteins oder eines Teils des Tat-Proteins in einer biologischen Probe, umfassend:
- das Inkontaktbringen der Probe mit anti-Tat-Antikörpern, welche durch das Ausführen des Verfahrens nach Anspruch 11 erhalten worden sind,
- die Bestimmung der Anwesenheit der Antigen-Antikörper-Komplexe.

13. Kit für die Diagnose einer Infektion durch HIV durch die Bestimmung der Anwesenheit des Tat-Proteins oder eines Teils des Tat-Proteins in einer biologischen Probe, umfassend:
- Reagenzien für die Bildung eines für die Antigen-Antikörper-Reaktion günstigen Mediums, umfassend Antikörper, welche durch das Ausführen des Verfahrens nach Anspruch 11 erhalten worden sind,
- Reagenzien, welche den Nachweis der Antigen-Antikörper-Komplexe erlauben,
- gegebenenfalls eine biologische Referenzprobe (negative Vergleichsprobe), welche kein Antigen enthält,
- gegebenenfalls eine biologische Referenzprobe (positive Vergleichsprobe), welche eine vorher festgelegte Menge Antigen enthält.

14. Verfahren zur Synthese einer Tat-Variante, wie in einem der Ansprüche 1 bis 4, 9 und 10 definiert, **dadurch gekennzeichnet, dass** es Schutzgruppen vom FMOC-Typ, vorzugsweise vom fastFMOC-Typ einsetzt.

## Claims

1. Anti-HIV-1 vaccine comprising an HIV-1 Tat protein comprising between 99 and 106 amino acids, capable of binding to TAR and incapable of transactivating, in combination with a pharmaceutically acceptable vehicle and, where appropriate, one or more appropriate immunity adjuvants.

2. Vaccine according to Claim 1, **characterized in that** it is a Tat whose nucleotide sequence has at least one mutation compared with the nucleotide sequence of a functional Tat.

3. Vaccine according to Claim 2, **characterized in that** the mutation affects at least one of the cysteines of the Tat protein.

4. Vaccine according to Claims 1 to 3, **characterized in that** it is the Tat of the HIV-1 Oyi variant.

5. Vaccine according to Claims 1 to 4, **characterized in that** it is a chemical synthetic Tat.

6. Vaccine according to Claim 5, **characterized in that** the Tat is synthesized in a solid phase.

7. Vaccine according to one of claims 1 to 4, **characterized in that** it is a recombinant Tat.

8. Anti-HIV vaccine, **characterized in that** it contains an expression vector comprising a nucleotide sequence coding for an HIV-1 Tat protein as defined in Claim 1, and the elements necessary for its expression.

9. Vaccine according to Claim 4, **characterized in that** it comprises at least the N-terminal region and/or the basic region of the Tat sequence.

10. Vaccine according to one of Claims 1 to 9, **characterized in that** the Arg-Gly-Asp region situated at the C-terminal end of Tat is modified.

11. Method for preparing anti-Tat polyclonal antibodies capable of recognizing several Tat variants, comprising:
- the immunization of an animal, except humans, by means of injecting a Tat protein as defined in Claim 5 combined with an immune response adjuvant,
- the purification of the specific antibodies contained in the serum of the immunized animals,
**characterized in that** the Tat protein used in the immunization step corresponds to the Oyi variant.

12. Method for detecting the presence of the Tat protein or of part of the Tat protein in a biological sample, comprising:
- bringing said sample into contact with anti-Tat antibodies obtained by implementing the method according to Claim 11,
- determining the presence of the antigen-antibody complexes.

13. Kit for the diagnosis of the HIV infection by determining the presence of the Tat protein or of part of the Tat protein in a biological sample, comprising:
- reagents for the preparation of a medium suitable for the antigen-antibody reaction, comprising antibodies obtained by carrying out the method according to Claim 11,
- reagents allowing the detection of the antigen-antibody complexes,
- optionally, a reference biological sample (negative control) free of antigen,
- optionally, a reference biological sample (positive control) containing a predetermined quantity of antigen.

14. Method for synthesizing a Tat variant as defined in one of Claims 1 to 4, 9 and 10, **characterized in that** it uses protective groups of the FMOC type, preferably of the fastFMOC type.
